# EUROPÄISCHE PATENTSCHRIFT

(11) **EP 2 585 230 B1**
(45) Veröffentlichungstag und Bekanntmachung des Hinweises auf die Patenterteilung: **11.03.2015**
(21) Anmeldenummer: 11729589.9
(22) Anmeldetag: 21.06.2011
(51) Int. Cl.: C07B 63/00, C07C 2/62, C07C 9/16, C23G 1/06, C23G 1/08, C23G 1/19

(54) **Reinigung von HF-Alkylierungsanlagen**
Purification of HF alkylation units
Nettoyage d'installations d'alkylation à l'acide fluorhydrique

(30) Priorität: 23.06.2010 DE 102010024781
(43) Veröffentlichungstag der Anmeldung: 01.05.2013
(73) Patentinhaber: Clean & Save - Systeme E. Mogge Beiz- Und Reinigungsservice für Industrieanlagen GmbH, 47877 Willich (DE)
(72) Erfinder: MONNINGER, Erich, 41069 Mönchengladbach (DE)
(74) Vertreter: Von Kreisler Selting Werner - Partnerschaft von Patentanwälten und Rechtsanwälten mbB
(86) Internationale Anmeldenummer: PCT/EP2011/060342
(87) Internationale Veröffentlichungsnummer: WO 2011/161103

(56) Entgegenhaltungen:
- DD-A7- 282 814
- EnvTech, Inc.: "Process Equipment Decontamination & Environmental Solutions", , 2002, Seiten 1-17, XP55018676, Gefunden im Internet: URL:http://envtech.com/downloads/general-i nfo/EnvTech-CB.pdf [gefunden am 2012-02-07]
- JAMES F HIMES ET AL: "Chapter 9 Gasoline components; 9.1 Motor fuel alkylation", 1. Januar 2006 (2006-01-01), HANDBOOK OF PETROLEUM PROCESSING, SPRINGER, PAGE(S) 355 - 416, XP009156319, Seite 355 Seiten 360-361 Seite 364, Absatz 2
- DOBIS, J.D. ET AL.: "The Top Ten Corrosion Issues Affecting HF Alkylation Units", CORROSION 2007 CONFERENCE & EXPO, Bd. Paper 07570, 2007, Seiten 1-19, XP009156313,

## Beschreibung

Gegenstand der Erfindung ist ein Verfahren zur Reinigung von HF-Alkylierungsanlagen und Teilen davon.

Aus EP 0989 106 A1 und dem hier genannten Stand der Technik ist grundsätzlich die Herstellung von Kohlenwasserstoffen im Benzinsiedebereich durch Oligomerisierung leichter Olefine und Sättigung der resultierenden schwereren Olefinen bekannt. Danach wird die Alkylierung von Isobutan, Propylenen, Butenen und Amylenen unter Verwendung eines Fluorwasserstoffsäurekatalysators (HF) als HF-Alkylierung bezeichnet.

In den Alkylierungsanlagen und Teilen davon setzten sich mit der Zeit verschiedenste Rückstände ab, die darüber hinaus mehr oder weniger große Mengen an Fluorwasserstoffsäure enthalten.

Je nach Größe und Bauart der Anlagen befinden sich beispielsweise 60 bis 120 Tonnen Flusssäure im Produktionsprozess. Die gesamte Anlage wird üblicherweise aus normalem C-Stahl gebaut. Während des Betriebes bildet sich eine Eisenfluoridschicht auf den Oberflächen, die aufgrund verschiedener Betriebszustände, Strömungsverhältnisse, Turbulenzen und so weiter abgewaschen wird und sich in strömungsungünstigen Zonen anlagert., So ist diese Anlagerung in Mantelseiten von Wärmetauschern teilweise so stark, dass diese zu einem geschlossenen "Eisenfluoridklumpen" verwachsen. Diese lassen sich dann im Reparatur- oder Revisionsfall nur noch sehr schwer ziehen oder zerlegen.

Üblicherweise wird im Reparatur- oder Revisionsfall die gesamte Anlage entleert und die Produktionsrückstände, wie auch die HF- Rückstände entfernt beziehungsweise neutralisiert.

Im Stand der Technik ist hierzu von einem Anlagenbetreiber empfohlen worden, die gesamt Anlage mit gasförmigem Ammoniak zu fluten, um die Flusssäure zu neutralisieren. Hierbei erreicht man allenfalls die freie Flusssäure, nicht jedoch die durch eingeschlossene Eisenfluoridbeläge in den Anlagen enthaltene Flusssäure. Diese stellt bei einem späteren Öffnen der Anlagen eine große Gefahr für das Reinigungspersonal dar. Zudem bleiben alle Inkrustierungen erhalten, so dass die Anlagen oder Anlagenteile nur sehr aufwändig und unter hoher Gefährdung vollständig zerlegbar sind. Hierbei müssen die gesamten Instandhaltungs- und Inspektionsarbeiten unter entsprechender persönlicher Schutzausrüstung des Reinigungspersonals durchgeführt werden. Alle Rückstände müssen als Sondermüll behandelt und unter entsprechend hohem Aufwand kostenintensiv beseitigt werden.

In einem anderen Fall des Standes der Technik wurde **von EnvTech, Inc. on "Process Equipment Decontamination and Environmental Solutions"** vorgeschlagen, die Anlagen mit einer alkalischen Mischung aus Natriumkarbonat, Natriumbikarbonat, Natriumglukonat, EDTA und Netzmittelzusätzen zu spülen. Hierbei wird eine verdünnte wässrige Lösung bei einer Temperatur nahe des Siedepunkts des Wassers eingesetzt. **Beschrieben ist hier eine Reinigung und Neutralisation einer HF-Alkylierungsanlage in einem Schritt, in welchem ein Puffersystem mit Komplexbildnern eingesetzt wird. An anderer Stelle wird ein Verfahren in drei Schritten beschrieben, in welchem eine Säure zur Entfernung von Polymeren und Ablagerungen eingesetzt wird. Nach dem Säurereinigungsschritt ist ein Spülschritt und ein Neutralisierungsschritt erforderlich.**

Bei dieser Reinigungsmethode kann das Eisenfluorid teilweise in Natriumfluorid umgewandelt werden, bleibt jedoch in seinem Volumen, insbesondere an Ort und Stelle erhalten. Damit sich die aufgeschlämmten Rückstände nicht erneut wieder absetzen, wird die vorgenannte Reinigungslösung möglichst schnell aus den Anlagen entfernt. Berücksichtigt man, dass üblicherweise jede der vorgenannten Anlagen ein sogenanntes Neutralisationsbecken aufweist, so kann allein die Leerung der Anlage bei einem Volumen von beispielsweise von 600 bis 1500 m³ mehrere Stunden dauern, wenn diese Neutralisationsbecken, wie in der Praxis üblich, nur etwa 50 m³ aufnehmen können. Zudem haben die Anlagen bauseitig nur sehr dünne Restentleerungsleitungen was eine schnelle Entleerung erschwert.

Das vorgenannte Verfahren hat darüber hinaus weitere Nachteile und Probleme, insbesondere bei Wärmetauschern, die zum Beispiel mantelseitig mit Eisenfluorid zugesetzt sind. Diese müssen von der Rohrseite her extern bedampft zum Aufheizen beaufschlagt werden. In der Zwischenzeit kann aber an ungünstigen Stellen durch die Reinigungslösung eine Vermischung von Flusssäure enthaltendem Wasser entstanden sein, welches beim Aufheizen insbesondere über 100°C überaus korrosiv sein kann und zu Rohrdurchbrüchen führen kann. Um die Inkrustierungen einigermaßen zu durchweichen ist es erforderlich, mit hohen Volumenströmen die Reinigungsflüssigkeit zu zirkulieren. Im vorgenannten Verfahren sollen hierfür die anlageneigenen Pumpen eingesetzt werden. Diese sind häufig jedoch nicht dafür ausgelegt und andererseits werden, da oft magnetgekuppelt von den nicht aufgelösten Abrasiva und Verschmutzungsrückständen zerstört.

Somit bleiben die Rückstände praktisch vollständig erhalten, wodurch dieselben Probleme bei den Revisionsarbeiten auftauchen, die durch die vorgenannte Ammoniakmethode erhalten werden. Allerdings fehlt hier die Beeinträchtigung durch das Ammoniakgas. Dem gegenüber ist jedoch die sehr problematische Abwasserbehandlung des eingesetzten EDTA zu berücksichtigen. Dem entsprechend besteht nach wie vor ein Bedürfnis zur verbesserten Reinigung von HF- Alkylierungsanlagen und Teilen davon, bei dem die Nachteile der oben genannten Verfahren überwunden werden.

In einer ersten Ausführungsform der vorliegenden Erfindung wird die vorgenannte Aufgabe gelöst durch ein Verfahren zur Reinigung von HF-Alkylierungsanlagen und Teilen davon **aus C-Stahl,** wobei man nach Beendigung der Reaktion
(a) die Anlagen oder Teile davon in einem ersten Schritt mit einer wässrigen Lösung einer **nichtoxidierenden** anorganischen und/oder organischen Säure in Kontakt bringt,
(b) in einem zweiten Schritt die gereinigten und gegebenenfalls neutralisierten Anlagen oder Teile davon mit einer wässrigen Lösung eines Komplexbildners für Eisenionen behandelt und
(c) anschließend in einem dritten Schritt die vorgenannten Anlagen oder Teile davon mit einer wässrigen Lösung **von Kalium**hydroxid neutralisiert oder überneutralisiert.

Mit Hilfe der vorliegenden Erfindung ist es möglich, die Eisenfluoridrückstände restlos aufzulösen und ohne Gefährdung für Material und Personal durchzuführen.

In einem ersten Reinigungsschritt werden daher die Anlagen oder Teile davon mit einer wässrigen Lösung einer anorganischen und/oder organischen Säure in Kontakt gebracht. Besonders bevorzugt eignet sich hierfür verdünnte, wässrige Salzsäure, beispielsweise in einer Konzentration von 3 bis 7 Gew.-%.

Die wässrige Lösung der anorganischen Säure sollte insbesondere einen Inhibitor aufweisen, wobei im Sinne der vorliegenden Erfindung Diethyl-Thioharnstoff eingesetzt wird. Dessen Konzentration beträgt beispielsweise 50 g/m³ Reinigungslösung. Dieser Inhibitor verhindert weitestgehend einen Angriff der Reinigungssäure wie auch der während der Reinigung freiwerdenden Flusssäure auf die Stahloberfläche.

Gegebenenfalls kann diese wässrige Lösung auch Lösungsvermittler und/oder Tenside enthalten. Als Lösevermittler kann beispielsweise Diethylenglykol eingesetzt werden. Als Netzmittel/Tensid kann beispielsweise eine verdünnte, wässrige Alkylbenzolsulfonsäure eingesetzt werden, wobei der Gesamtgehalt an Tensid in der Größenordnung von 0,1 Gew.-%, bezogen auf die Reinigungslösung, eingesetzt wird.

Im Unterschied zum Stand der Technik kann dieser erste Reinigungsschritt bei einer Temperatur oberhalb des Gefrierpunkts der Reinigungslösung eingesetzt werden, so dass ein Aufheizen nicht erforderlich ist. Dennoch ist es im Sinne der vorliegenden Erfindung selbstverständlich möglich, auch in diesem ersten Reinigungsschritt bei einer gegenüber dem Gefrierpunkt der Reinigungslösung erhöhten Temperatur zu arbeiten.

Mit Hilfe dieser sauren Reinigungslösung werden die in der Anlage oder den Teilen befindlichen Rückstände chemisch aufgelöst. Beim Einsatz von nichtoxidierenden Säuren bilden sich daher aus den Eisenfluoriden die entsprechenden Salze, beispielsweise Eisen(II)-chlorid, wodurch die entsprechende Menge an Flusssäure wieder freigesetzt wird.

Somit wird die Reinigungslösung insbesondere mittels externer Pumpen, Ventilen, Schläuchen durch das System im Zwangskreislauf geführt und permanent durch eine Reihe von Analysen überwacht.

Hierzu zählen beispielsweise die Titration der Säure gegen Maßlösung Natronlauge; der Test der Wirkungsweise des Inhibitors mit Stahlwolle und die Bestimmung der Eisenkonzentration in der Reinigungslösung, die photometrisch oder durch Titration/ Filtration erfolgen kann. Geht bei genügendem Angebot an freier Säure kein weiteres Eisen mehr in Lösung, ist dies der Nachweis, dass sämtliches Eisenfluorid gelöst und die Reinigung beendet ist. Die Lösung wird dann möglichst unter Stickstoffüberdruck entleert und extern, beispielsweise mit Calciumhydroxid/Kalkmilch, neutralisiert **(nicht erfindungsgemäß).** Die Flusssäure fällt hierbei als Calciumfluorid aus und kann gefahrlos abgepresst und deponiert oder mit als Ausgangspunkt für die Herstellung von Flusssäure verwendet werden.

Nach der vorgenannten Säurebehandlung des ersten Verfahrensschrittes ist die Oberfläche der Anlagen oder Anlagenteile äußerst aktiv. Dementsprechend kann eine passivierende Nachbehandlung mit einem Komplexbildner für Eisenionen erfolgen. So ist es möglich, das System mit einer stark verdünnten, wässrigen, organischen mehrwertigen Säure zu spülen, wobei hier gegebenenfalls 0,1 bis 5 Gew.-% einer wässrigen Citronensäure verwendet werden kann. Insbesondere sollte hiermit zunächst die noch in den Tiefpunkten der Anlage verbleibende Reinigungslösung verspült werden. Bevorzugt im Sinne der vorliegenden Erfindung wird diese verdünnte, wässrige, organische Säurelösung im Verlauf von 4 bis 6 Stunden in den Anlagen zirkuliert.

In einem notwendigen wesentlichen dritten Schritt der vorliegenden Erfindung werden die vorgenannten Anlagen oder Teile davon mit einer wässrigen Lösung eines **Kalium**hydroxid neutralisiert oder überneutralisiert. Beispielsweise ist es hier möglich, mit verdünnter, wässriger Kalilauge bis zu einem pH-Wert von 9 bis 10 zu überalkalisieren und anschließend die Oberfläche erneut zu passivieren. Hierfür bietet sich beispielsweise eine verdünnte, wässrige Lösung von Wasserstoffperoxid, insbesondere 0,1 bis 5 Gew.-% in Wasser an. Hierbei bildet sich ein Eisenkomplex in der Anlage beziehungsweise auf den Oberflächen der Teile davon, der keinen Neutralisationsschlamm wie bei der Verwendung von Natronlauge hinterlässt.

Während üblicherweise der Alkalisierungsschritt durch Ammoniaklösung durchgeführt wird, führt das erfindungsgemäß eingesetzte Alkalimetall- und/oder Erdalkalimetallhydroxid im vorliegenden Fall in den Abwasseranlagen aufgrund des geringen beziehungsweise nicht vorhandenen Stickstoffgehalts nicht zu Problemen.

Bei Anlagen, die erfahrungsgemäß wenig Eisenfluorid bilden, kann im ersten Schritt vorzugsweise anstelle der anorganischen nicht oxidiativen Säure auch die organische Säure eingesetzt und somit die Reinigung insgesamt verkürzt werden. Hierbei ist es beispielsweise möglich, mit inhibierter, verdünnter, wässriger Citronensäure, beispielsweise einer Konzentration von 3 bis 5 Gew.-% in die Anlage einzubringen, ohne diese vollständig zu füllen. Vorzugsweise wird diese verdünnte, wässrige, organische Säure jedoch in die Anlagenvolumina eingebracht, welche die meiste Eisenfracht mit sich führt und dementsprechend möglichst schnell ausschleust und ohne Zirkulation innerhalb der Anlage. In den darauf folgenden Arbeitsschritten kann dann die Zirkulation wie üblich durchgeführt werden.

## Patentansprüche

1. Verfahren zur Reinigung von HF-Alkylierungsanlagen und Teilen davon aus C-Stahl, wobei man nach Beendigung der Reaktion
(a) die Anlagen oder Teile davon in einem ersten Schritt mit einer wässrigen Lösung einer nicht oxidierenden anorganischen und/oder organischen Säure in Kontakt bringt,
(b) in einem zweiten Schritt die gereinigten und gegebenenfalls neutralisierten Anlagen oder Teile davon mit einer wässrigen Lösung eines Komplexbildners für Eisenionen behandelt,
(c) anschließend in einem dritten Schritt die vorgenannten Anlagen oder Teile davon mit einer wässrigen Lösung von Kaliumhydroxid neutralisiert oder überneutralisiert.

2. Verfahren nach Anspruch 1, **dadurch gekennzeichnet, dass** die wässrige Lösung einer anorganischen und/oder organischen Säure Citronensäure oder Chlorwasserstoff enthält.

3. Verfahren nach Anspruch 1 oder 2, **dadurch gekennzeichnet, dass** die wässrige Lösung einer anorganischen und/oder organischen Säure 2 bis 10 Gew. % der Säure enthält.

4. **Verfahren nach Anspruch 3, dadurch gekennzeichnet, dass die wässrige Lösung 3 bis 7 Gew. % der Säure enthält.**

5. Verfahren nach Anspruch 2, **dadurch gekennzeichnet, dass** die wässrige Chlorwasserstoff-Lösung weiterhin Beizinhibitor und/oder Tensid enthält.

6. Verfahren nach einem der Ansprüche 1 bis 5, **dadurch gekennzeichnet, dass** die wässrigen Lösung des Kaliumhydroxids einen Kaliumhydroxidgehalt von 20 bis 50 Gew. % aufweist.

7. **Verfahren nach Anspruch 6, dadurch gekennzeichnet, dass die wässrige Lösung des Kaliumhydroxids einen Kaliumhydroxidgehalt von 30 bis 35 Gew. % aufweist.**

8. Verfahren nach einem der Ansprüche 1 bis 7, **dadurch gekennzeichnet, dass** man eine verdünnte wässrige Lösung eines Komplexbildners einsetzt, die Citronensäure und/ oder Weinsäure einsetzt.

9. Verfahren nach einem der Ansprüche 1 bis 8, **dadurch gekennzeichnet, dass** man eine verdünnte wässrige Lösung eines Komplexbildners mit einer Konzentration des Komplexbildners von 0,1 bis 10 Gew. % einsetzt.

10. **Verfahren nach Anspruch 9, dadurch gekennzeichnet, dass man eine verdünnte wässrige Lösung eines Komplexbildners mit einer Konzentration des Komplexbildners von 0,1 bis 5 Gew. % einsetzt.**

## Claims

1. A process for cleaning HF alkylation plants and parts thereof made of carbon steel, wherein, after the reaction is complete,
(a) in a first step, the plants or parts thereof are contacted with an aqueous solution of a non-oxidizing inorganic and/or organic acid;
(b) in a second step, the cleaned and optionally neutralized plants or parts thereof are treated with an aqueous solution of a complexing agent for iron ions;
(c) subsequently, in a third step, the above mentioned plants or parts thereof are neutralized or overneutralized with an aqueous solution of potassium hydroxide.

2. The process according to claim 1, **characterized in that** said aqueous solution of an inorganic and/or organic acid contains citric acid or hydrogen chloride.

3. The process according to claim 1 or 2, **characterized in that** said aqueous solution of an inorganic and/or organic acid contains from 2 to 10% by weight of said acid.

4. The process according to claim 3, **characterized in that** said aqueous solution contains from 3 to 7% by weight of said acid.

5. The process according to claim 2, **characterized in that** said aqueous hydrogen chloride solution further contains a pickling inhibitor and/or a surfactant.

6. The process according to any of claims 1 to 5, **characterized in that** said aqueous solution of potassium hydroxide has a potassium hydroxide content of from 20 to 50% by weight.

7. The process according to claim 6, **characterized in that** said aqueous solution of potassium hydroxide has a potassium hydroxide content of from 30 to 35% by weight.

8. The process according to any of claims 1 to 7, **characterized in that** a diluted aqueous solution of a complexing agent using citric acid and/or tartaric acid is employed.

9. The process according to any of claims 1 to 8, **characterized in that** a diluted aqueous solution of a complexing agent with a concentration of the complexing agent of from 0.1 to 10% by weight is employed.

10. The process according to claim 9, **characterized in that** a diluted aqueous solution of a complexing agent with a concentration of the complexing agent of from 0.1 to 5% by weight is employed.

## Revendications

1. Procédé pour nettoyer des usines d'alkylation HF et de parties de celles-ci, qui sont en acier au carbone, dans lequel, après l'achèvement de la réaction,
(a) lesdites usines ou parties de celles-ci sont mises en contact avec une solution aqueuse d'un acide inorganique et/ou organique, non-oxydant, dans une première étape,
(b) les usines ou parties de celles-ci, nettoyées et éventuellement neutralisées, sont traitées avec une solution aqueuse d'un agent complexant pour les ions de fer, dans une deuxième étape,
(c) ensuite, les usines ou leur parties susmentionnées sont neutralisées ou surneutralisées avec une solution aqueuse d'hydroxyde de potassium, dans une troisième étape.

2. Procédé selon la revendication 1, **caractérisé en ce que** ladite solution aqueuse d'un acide inorganique et/ou organique contient de l'acide citrique ou du chlorure d'hydrogène.

3. Procédé selon la revendication 1 ou 2, **caractérisé en ce que** ladite solution aqueuse d'un acide inorganique et/ou organique contient de 2 à 10 % en poids dudit acide.

4. Procédé selon la revendication 3, **caractérisé en ce que** ladite solution aqueuse contient de 3 à 7 % en poids dudit acide.

5. Procédé selon la revendication 2, **caractérisé en ce que** ladite solution aqueuse de chlorure d'hydrogène contient en outre un inhibiteur de décapage et/ou un agent tensioactif.

6. Procédé selon l'une quelconque des revendications 1 à 5, **caractérisé en ce que** ladite solution aqueuse d'hydroxyde de potassium présente une teneur en hydroxyde de potassium de 20 à 50% en poids.

7. Procédé selon la revendication 6, **caractérisé en ce que** ladite solution aqueuse d'hydroxyde de potassium présente une teneur en hydroxyde de potassium de 30 à 35% en poids.

8. Procédé selon l'une quelconque des revendications 1 à 7, **caractérisé en ce qu'**une solution aqueuse diluée d'un agent complexant utilisant de l'acide citrique et/ou de l'acide tartrique est utilisée.

9. Procédé selon l'une quelconque des revendications 1 à 8, **caractérisé en ce qu'**une solution aqueuse diluée d'un agent complexant présentant une teneur en ledit agent complexant de 0,1 à 10 % en poids est utilisée.

10. Procédé selon la revendication 9, **caractérisé en ce qu'**une solution aqueuse diluée d'un agent complexant présentant une teneur en ledit agent complexant de 0,1 à 5 % en poids est utilisée.
